(19) Europäisches Patentamt
European Patent Office
Office européen des brevets

(11) **EP 3 591 032 A1**

(12) **EUROPEAN PATENT APPLICATION**
published in accordance with Art. 153(4) EPC

(43) Date of publication:
**08.01.2020 Bulletin 2020/02**

(51) Int Cl.:
*C12M 1/34* *(2006.01)*    *C12M 1/00* *(2006.01)*
*G01N 33/48* *(2006.01)*    *G01N 33/49* *(2006.01)*
*G06T 7/00* *(2017.01)*

(21) Application number: **18775175.5**

(22) Date of filing: **20.02.2018**

(86) International application number:
**PCT/JP2018/005946**

(87) International publication number:
**WO 2018/180012 (04.10.2018 Gazette 2018/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(30) Priority: **31.03.2017   JP 2017072849**

(71) Applicant: **Sony Corporation
Tokyo 108-0075 (JP)**

(72) Inventor: **NAKAGAWA Kazuhiro
Tokyo 108-0075 (JP)**

(74) Representative: **D Young & Co LLP
120 Holborn
London EC1N 2DY (GB)**

(54) **INFORMATION PROCESSING DEVICE, INFORMATION PROCESSING SYSTEM, AND INFORMATION PROCESSING METHOD**

(57)    The present technology provides a technique capable of evaluating an intracellular metabolic state in a non-stained state.

In this regard, the present technology provides, for example, an information processing device including at least an analysis unit for calculating a morphological change amount of a cell per unit time from an image obtained by imaging the cell, and evaluating an intracellular metabolic state on the basis of the morphological change amount of the cell per unit time.

## FIG. 1

**Description**

TECHNICAL FIELD

The present technology relates to an information processing device, an information processing system, and an information processing method. More specifically, the present technology relates to an information processing device, an information processing system, and an information processing method capable of evaluating an intracellular metabolic state in a non-stained state.

BACKGROUND ART

In recent years, due to production of an iPS cell, technological advances in fields of regenerative medicine, cell therapy, tissue engineering, and cell engineering have become remarkable, and demands for evaluating the state of a cell and evaluating an effect and an influence of a drug or the like using a cell have also become very large. Here, in the fields of regenerative medicine and cell therapy, there is a reality that strict quality control is required in order to use a cell as a preparation. In quality control of a cell, in particular, it is very important to measure an intracellular metabolic state and viability. In this case, it is naturally assumed that a quality-controlled cell is returned to a human body, and therefore a nondestructive, noninvasive, and non-staining evaluation method is desired.

Furthermore, also in a case where a therapeutic effect of a drug or the like on a disease using a cell and a tissue produced by tissue engineering or cell engineering is examined, in order to find the state of a cell, it is very important to evaluate an intracellular metabolic state and viability which are basic elements of the state of a cell. In particular, in a case where a cytotoxic activity of an anticancer agent or the like is examined, an intracellular metabolic state and viability are direct evaluation indices. Also in these in vitro tests using a cell and a tissue, a nondestructive, noninvasive, and non-staining evaluation method is required from viewpoints of a change over time and an interaction between a drug and other molecules.

Generally, as a method for evaluating an intracellular metabolic state and viability, a method for disrupting a cell and measuring the amount of ATP and a method for performing measurement using a substance which is conjugated with a metabolic enzyme in a cell and changes an absorption spectrum thereof are known. Furthermore, there is also a method for introducing a substance that responds to an oxygen concentration or pH change into a cell culture solution or a cell and performing evaluation. However, these are methods requiring cell disruption, staining, and a foreign substance. Therefore, in a case where a method using staining and a foreign reagent is performed in a drug toxicity test, an anticancer agent cytotoxicity test, a cytotoxicity test with an immune cell, or the like, there may be a difference in the uptake amount of a staining agent, the foreign reagent, or the like depending on the state of a cell.

Here, Patent Document 1 discloses, as a method for evaluating an intracellular metabolic state in a non-staining manner, a method for quantifying movement of an intracellular structure to perform evaluation. This method is an effective method in a case where an intracellular structure is clearly observed in non-staining imaging such as phase contrast image. Specifically, for example, this method is useful in observation in a culture state in which cells adhere, such as monolayer culture.

CITATION LIST

PATENT DOCUMENT

Patent Document 1: Japanese Patent Application Laid-Open No. 2017-16628

SUMMARY OF THE INVENTION

PROBLEMS TO BE SOLVED BY THE INVENTION

Under such a circumstance, further development of a technique capable of evaluating an intracellular metabolic state in a non-stained state is desired.

Therefore, a main object of the present technology is to provide a technique capable of evaluating an intracellular metabolic state in a non-stained state.

SOLUTIONS TO PROBLEMS

First, the present technology provides an information processing device including at least an analysis unit for calculating the morphological change amount of a cell per unit time from an image obtained by imaging the cell, and evaluating an intracellular metabolic state on the basis of the morphological change amount of the cell per unit time.

In the information processing device according to the present technology, the morphological change amount may be based on the area change amount of the cell per unit time.

Furthermore, in the information processing device according to the present technology, the morphological change amount may be based on the change amount of the peripheral length of the cell per unit time.

Moreover, in the information processing device according to the present technology, the morphological change amount may be based on the change amount of the roundness of the cell per unit time.

In addition, in the information processing device according to the present technology, the morphological change amount may be based on the change amount of the volume of the cell per unit time.

Furthermore, in the information processing device according to the present technology, the morphological change amount may be based on the change amount of movement of a structure generated by the cell per unit time.

Moreover, in the information processing device according to the present technology, the analysis unit may further calculate the motion amount of the intracellular structure per unit time from an image obtained by imaging the cell, and may use the motion amount of the intracellular structure per unit time in the evaluation.

In addition, in the information processing device according to the present technology, the analysis unit may further color-code the inside of each cell according to a result of the evaluation. In this case, the analysis unit may further quantify distribution of the cell after the color-coding. Furthermore, in this case, the analysis unit may further extract a specific population from the cell after the color-coding.

Furthermore, in the information processing device according to the present technology, the analysis unit may calculate an intracellular ATP concentration on the basis of the morphological change amount of the cell per unit time.

Moreover, in the information processing device according to the present technology, the analysis unit may further set a threshold to a value of the evaluation to determine whether the cell is a living cell or a dead cell.

In addition, in the information processing device according to the present technology, the cell may be a floating cell. In this case, the floating cell may include at least one selected from the group consisting of a white blood cell, a white blood cell precursor cell, a white blood cell-derived tumor cell, and a blood circulating tumor cell.

Furthermore, the present technology also provides an information processing system including at least an imaging device and an information processing device including at least an analysis unit for calculating the morphological change amount of a cell per unit time from an image obtained by imaging the cell with the imaging device, and evaluating an intracellular metabolic state on the basis of the morphological change amount of the cell per unit time.

Moreover, the present technology provides an information processing method including at least an analysis procedure for calculating the morphological change amount of a cell per unit time from an image obtained by imaging the cell, and evaluating an intracellular metabolic state on the basis of the morphological change amount of the cell per unit time.

In the information processing method according to the present technology, in the analysis procedure, the motion amount of the intracellular structure per unit time may be further calculated from an image obtained by imaging the cell, and the motion amount of the intracellular structure per unit time may be used in the evaluation.

Note that in the present technology, "cell" is a concept including not only one cell but also a so-called "cell group" which is an assembly of a plurality of cells.

EFFECTS OF THE INVENTION

According to the present technology, an intracellular metabolic state can be evaluated in a non-stained state. Note that the effects described here are not necessarily limited, and may be any of the effects described in the present disclosure.

BRIEF DESCRIPTION OF DRAWINGS

Fig. 1 is a schematic diagram illustrating an example of a configuration of an information processing system 1 according to the present technology.

Fig. 2 is a schematic diagram illustrating an example of a hardware configuration of an information processing device 12 according to the present technology.

Fig. 3 is a process chart illustrating an information processing example 1 in an information processing method according to the present technology.

Fig. 4 is a process chart illustrating an information processing example 2 in the information processing method according to the present technology.

Fig. 5 is an image illustrating cell boundary recognition and tracking according to Example 1.

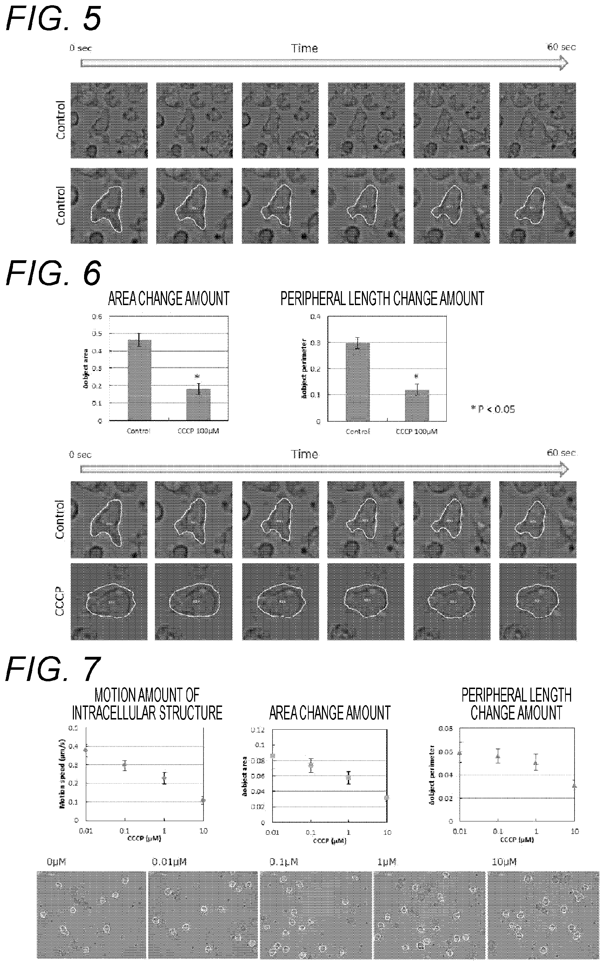

The upper drawing of Fig. 6 is a drawing-substituting graph illustrating an area change amount and a peripheral length change amount according to Example 1, and the lower drawing of Fig. 6 is an image illustrating cell boundary recognition and tracking in Control and in a case where CCCP is added according to Example 1.

The upper drawing of Fig. 7 is a drawing-substituting graph illustrating the motion amount (vector analysis), the area change amount, and the peripheral length change amount of an intracellular structure according to Example 2, and the lower drawing of Fig. 7 is a color map of a cell including CCCP according to Example 2.

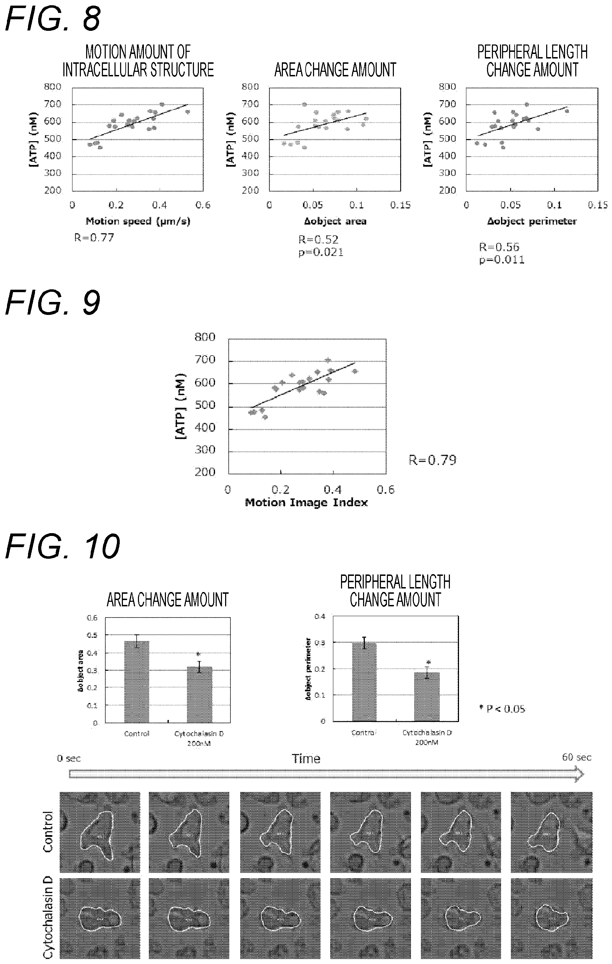

Fig. 8 is a drawing-substituting graph illustrating the motion amount (vector analysis), the area change amount, and the peripheral length change amount of an intracellular structure according to Example 3.

Fig. 9 is a drawing-substituting graph illustrating a correlation between an ATP concentration and a Motion Image Index according to Example 3.

The upper drawing of Fig. 10 is a drawing-substituting graph illustrating an area change amount and a peripheral length change amount according to Example 4, and the lower drawing of Fig. 10 is an image illustrating cell boundary recognition and tracking in Control and in a case where Cytochalasin D is added according to Example 4.

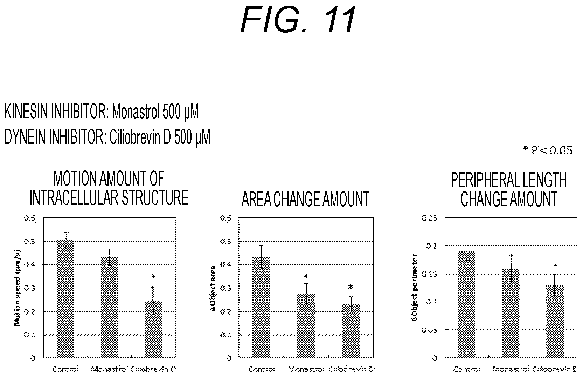

Fig. 11 is a drawing-substituting graph illustrating the motion amount (vector analysis), the area change amount, and the peripheral length change amount of an intracellular structure according to Example 5.

MODE FOR CARRYING OUT THE INVENTION

Hereinafter, preferred embodiments for implementing the present technology will be described with reference to the drawings. The embodiments described below exemplify representative embodiments of the present technology, and the scope of the present technology is not narrowly interpreted by the embodiments. Note that the description will be made in the following order.

    1. Information processing system 1

        (1) Imaging device 11
        (2) Information processing device 12

    2. Information processing device 12

        (1) Analysis unit 121
        (2) Imaging control unit 122
        (3) Image acquisition unit 123
        (4) Display control unit 124
        (5) Hardware configuration of information processing device 12

    3. Information processing method

        (1) Information processing example 1
        (2) Information processing example 2

1. Information processing system 1

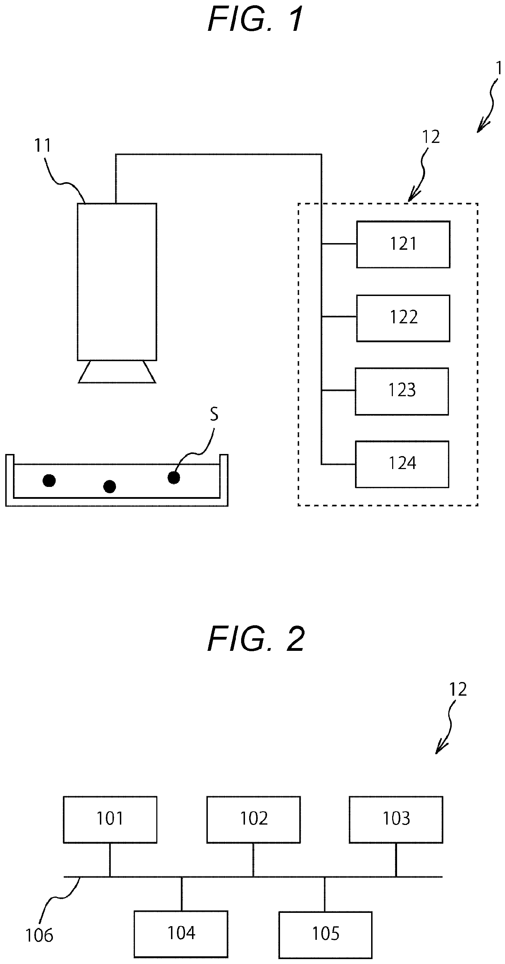

Fig. 1 is a schematic diagram illustrating an example of a configuration of an information processing system 1 according to the present technology. The information processing system 1 according to the present technology includes at least an imaging device 11 and an information processing device 12 described later. Furthermore, the information processing system 1 may include another device as necessary. Note that Fig. 1 also illustrates a cell S to be observed, but the cell S is not necessarily included in the present technology. Hereinafter, each device will be described in detail.

(1) Imaging device 11

The imaging device 11 can be a device capable of imaging the cell S to be observed over time to generate an image. Specifically, for example, the imaging device 11 can be a microscope including a microscope optical system or an image sensor, or the like, and may be a device capable of imaging an image (still image) at a predetermined imaging interval (for example, 1 frame/second or more) or a device capable of imaging continuous images (moving images). Furthermore, the imaging device 11 can receive control on an imaging range and an imaging interval by the information processing device 12 described later.

The imaging range of the imaging device 11 is not particularly limited, and can be freely set appropriately, for example, can be set to a range including one cell or a range including a cell group. A photographing method of the imaging device 11 is not particularly limited, and only needs to be an optical photographing method capable of acquiring an image in a non-staining manner, such as bright field photography, dark field photography, phase difference photography, fluorescence photography, absorbed light imaging, or scattered light imaging. Hereinafter, an image photographed by the imaging device 11 is referred to as an "imaged image".

(2) Information processing device 12

The information processing device 12 can be a device capable of acquiring and processing an imaged image photographed by the imaging device 11. Specifically, for example, the information processing device 12 can be a personal computer or the like. Note that, in the present technology, the information processing device 12 may be formed integrally with the imaging device 11, or may be a device independent of the imaging device 11. Furthermore, the imaging device 11 may be connected to the information processing device 12 via a network. The information processing device 12 will be described in detail in "2. Information processing device 12".

In the present technology, a cell is not particularly limited, and any cell can be observed. Among cells, in particular, a floating cell can be used. The floating cell does not extend in an x direction or a y direction, but may have a thickness in a z direction. Therefore, the floating cell is not clearly observed in some cases as compared with a cell having an intracellular structure including an adherent cultured cell. Therefore, in a conventional method for quantifying movement of an intracellular structure, it is difficult to observe the floating cell having such a property. However, the present technology uses the morphological change amount of a cell as described later, and therefore can evaluate an intracellular metabolic state with high accuracy even in a case of such a floating cell.

The floating cell is not particularly limited, but preferably includes at least one selected from the group consisting of a white blood cell, a white blood cell precursor cell, a white blood cell-derived tumor cell, and a blood circulating tumor cell.

2. Information processing device 12

The information processing device 12 according to the present technology includes at least an analysis unit 121. Furthermore, as illustrated in Fig. 1, the information processing device 12 may include other parts such as an imaging control unit 122, an image acquisition unit 123, and a display control unit 124 as necessary. Note that each part will be described in detail below.

(1) Analysis unit 121

The analysis unit 121 calculates a morphological change amount of a cell per unit time from an image obtained by imaging the cell, and evaluates an intracellular metabolic state on the basis of the morphological change amount of the cell per unit time.

Specifically, the analysis unit 121 recognizes a boundary between a cell area and a non-cell area in each cell or cell group present in a range designated by a user. Then, the analysis unit 121 tracks the boundary in the cell or the cell group in continuous imaged images, or recognizes the boundary in each imaged image. Thereafter, the analysis unit 121 calculates the morphological change amount from the tracked boundary in the cell or the cell group or the recognized boundary in the cell or the cell group. Note that the analysis unit 121 may recognize the boundary between a cell area and a non-cell area in each cell or cell group present in the entire imaged image.

The morphological change amount can be based on a change amount of, for example, the area of the cell, the peripheral length, the roundness of the cell, the volume of the cell, movement of a structure generated by the cell (for example, protrusion, temporary foot, or the like), or the like per unit time. Note that in the present technology, the morphological change amount can be determined using an average value, a median value, a maximum value, a standard deviation, and the like of these indices in a continuous image.

Specifically, for example, as illustrated in Example 1 and the like described later, the average of the area of a cell, the average of the peripheral length of the cell, and the change amounts thereof in a continuous image are calculated, and a change amount with respect to the average of the area or the peripheral length can be calculated as the morphological change amount (area change amount or peripheral length change amount).

Then, the analysis unit 121 can evaluate the intracellular metabolic state on the basis of the morphological change amount of the cell per unit time.

Specifically, the analysis unit 121 can execute image recognition processing on an imaged image, and can evaluate the intracellular metabolic state using the morphological change amount of the cell and a result of the image recognition processing. For example, the analysis unit 121 can detect a cell, a cell group, and the like included in an imaged image by pattern matching processing between an imaged image and a reference image to a morphological change of a cell, learning system image recognition processing, image recognition processing by luminance value distribution, and the like. Note that specification of a cell may be performed on the basis of designation of a range on an imaged image by a user.

Then, the analysis unit 121 can calculate the morphological change amount of a cell per unit time for each of cells detected by image recognition processing or designation by a user. For example, if it is assumed that a specific cell C is recognized in a specific imaged image G, the analysis unit 121 can calculate the morphological change amount only from a morphological change observed in an area corresponding to the range of the cell C in the imaged image G.

In the present technology, an evaluation index of the intracellular metabolic state is not particularly limited, but may be an intracellular ATP concentration. In other words, the analysis unit 121 can calculate the intracellular ATP concentration on the basis of the morphological change amount of a cell per unit time. This is based on the following fact as illustrated in Example 3 described later. That is, the morphology of a cell per unit time changes depending on the ATP concentration, and the morphological change amount of the cell per unit time also changes accordingly. As a result, the intracellular ATP concentration can be indirectly calculated on the basis of the morphological change amount, and the intracellular metabolic state can be evaluated in a non-staining manner.

Specifically, the analysis unit 121 can calculate the morphological change amount of each cell per unit time, and can indirectly calculate the ATP concentration on the basis of a correlation with the ATP concentration. In other words, many cells tend to have smaller values of morphological change amounts per unit time as the ATP concentration is lower. Therefore, when a value of the morphological change amount per unit time is small, it can be indirectly estimated that the ATP concentration is low.

Furthermore, the analysis unit 121 can calculate the ATP concentration by referring to the morphological change amount of a cell per unit time, specified in advance for each cell type (for example, a floating cell or the like, more specifically, a white blood cell, a white blood cell precursor cell, a white blood cell-derived tumor cell, a blood circulating tumor cell, or the like). Specifically, the analysis unit 121 can sequentially acquire the morphological change amount per unit time at a specific time interval, and can calculate the ATP concentration from a relative change amount thereof.

In the present technology, the analysis unit 121 can further set a threshold to a value of the evaluation and can determine whether the cell is a living cell or a dead cell. Specifically, for example, the analysis unit 121 can set a threshold to the intracellular ATP concentration, and can determine that the cell is a living cell if the concentration is equal to or higher than the threshold, and can determine that the cell is a dead cell if the concentration is lower than the threshold. This makes it possible to confirm viability of the cell using the present technology.

Furthermore, in the present technology, the analysis unit 121 can further color-code the inside of each cell according to a result of the evaluation to visualize the intracellular metabolic state. Color-coding can be performed, for example, by dividing the intracellular ATP concentration into stages, setting the respective stages to different colors, and the like. Furthermore, a portion to be color-coded is not particularly limited, and examples thereof include a boundary line between a cell area and a non-cell area in a cell or a cell group, a part or the whole of a cell or a cell group, and the like.

Then, the color-coded visualized image is output to a display or the like, displayed in color, and presented to a user. The visualized image may be generated for a plurality of imaged images having different imaging times, or may be generated by extracting only an individual cell or cell group specified by the analysis unit 121. This allows a user to evaluate an intracellular metabolic state with reference to this image.

The analysis unit 121 can further quantify the distribution of the cell after the color-coding. This quantification can be performed using, for example, a histogram, a whisker box plot, or the like. This improves usability.

Furthermore, the analysis unit 121 can further extract a specific population from the cell after the color-coding. The specific population can be, for example, a population satisfying conditions set in advance by the analysis unit 121 or a user. This improves usability. Note that in the present technology, after extracting the specific population, the analysis unit 121 can mask the population and display an image.

Note that in the present technology, not only the intracellular metabolic state in the analysis unit 121 but also the morphological change amount can be visualized by color-coding or the like. Color-coding can be performed, for example, by dividing values of the morphological change amount into stages, setting the respective stages to different colors, and the like. Furthermore, a portion to be color-coded is not particularly limited, and examples thereof include a boundary line between a cell area and a non-cell area in a cell or a cell group, a part or the whole of a cell or a cell group, and the like.

Note that in the present technology, the visualization of the intracellular metabolic state or the morphological change amount is not limited to the method performed by color-coding as described above, and may be any method capable of performing visualization for a user.

In the present technology, the analysis unit 121 further calculates the motion amount of the intracellular structure (for example, a granule, a fiber, or the like) per unit time from an image obtained by imaging the cell, and can use the motion amount of the intracellular structure per unit time in the evaluation. This makes it possible to more accurately evaluate a change in intracellular state.

Specifically, for example, at least two imaged images (frames) having different imaging times are compared, and the motion amount of the intracellular structure is calculated. In this case, the analysis unit 121 can calculate the motion amount at a resolution (5 μm or less) that can separate the intracellular structure.

More specifically, the analysis unit 121 extracts a motion vector between at least two imaged images having different imaging times. For example, the analysis unit 121 can extract a motion vector from a range designated by a user using a block matting method, a gradient method, or the like. Note that the analysis unit 121 may extract a motion vector from the entire imaged image.

The length of a motion vector is a motion amount. The analysis unit 121 can calculate a motion amount (motion speed) of an intracellular structure per unit time from an interval (time) of the two imaged images from which the motion vector has been extracted. Note that in the present technology, the motion amount of the intracellular structure can be determined using an average value, a median value, a maximum value, a standard deviation, and the like of the lengths of the motion vectors. Furthermore, the analysis unit 121 does not necessarily have to extract a motion vector, and may calculate a motion amount per unit time by another method.

Then, the analysis unit 121 can evaluate the intracellular metabolic state on the basis of the motion amount of the intracellular structure per unit time.

Specifically, the analysis unit 121 can execute image recognition processing on an imaged image, and can evaluate the intracellular metabolic state using the motion amount of the intracellular structure and a result of the image recognition processing. For example, the analysis unit 121 can detect a cell, a cell group, and the like included in an imaged image by pattern matching processing between an imaged image and a reference image to an intracellular structure (for example, a granule, a fiber, or the like), learning system image recognition processing, image recognition processing by luminance value distribution, and the like. Note that specification of a cell may be performed on the basis of designation of a range on an imaged image by a user.

Then, the analysis unit 121 can calculate the motion amount of the intracellular structure per unit time for each of cells detected by image recognition processing or designation by a user. For example, if it is assumed that a specific cell C is recognized in a specific imaged image G, the analysis unit 121 can calculate the motion amount only from a motion vector extracted from an area corresponding to the range of the cell C in the imaged image G.

Then, for example, as illustrated in the following numerical formula (1), by calculating a coefficient correlating with a change in intracellular state of a cell as a target (for example, ATP concentration and the like) in advance, and performing weighting with each correlation coefficient, a Motion image index can be calculated from the morphological change amount and the motion amount of the intracellular structure, and can evaluate the change in intracellular state using a result of the calculation.

[Numerical Formula 1]

$$\text{Motion image index} = \alpha \times \text{morphological change amount} + \beta \times \text{motion amount of intracellular structure} \quad \ldots (1)$$

α: Correlation coefficient of morphological change amount
β: Correlation coefficient of motion amount of intracellular structure

Note that in the above numerical formula (1), one parameter is used as the morphological change amount or the motion amount of the intracellular structure. However, the present technology is not limited thereto, and a plurality of such parameters may be used. For example, in a case where two parameters are used as the morphological change amount, a Motion image index is calculated by calculation according to the following numerical formula (2), and a change in intracellular state can be evaluated using a result of the calculation.

[Numerical Formula 2]

$$\text{Motion image index} = \alpha 1 \times \text{morphological change amount } 1 + \alpha 2 \times \text{morphological change amount } 2 + \beta \times \text{motion amount of intracellular structure} \quad \ldots (2)$$

α1: Correlation coefficient of morphological change amount 1
α2: Correlation coefficient of morphological change amount 2
β: Correlation coefficient of motion amount of intracellular structure

As a specific example of the above numerical formula (2), for example, the morphological change amount 1 can be an area change amount of a cell, the morphological change amount 2 can be a peripheral length change amount of a cell, and the motion amount of an intracellular structure can be an average of the lengths of intracellular motion vectors.

Note that in the information processing device 12 according to the present technology, as illustrated in information processing examples 1 and 2 described later, it is possible to intermittently repeat a process from a step of imaging an imaged image to a step of visualizing a change in intracellular state. This makes it possible to confirm a fluctuation of a metabolic state of each cell over time.

As described above, in the present technology, by evaluating a metabolic state and viability of a cell in a non-staining manner, the cell can be continuously measured, which is useful for quality control of the cell. Furthermore, a cytotoxic activity can be evaluated without depending on uptake or concentration distribution of a staining agent or a foreign reagent into a cell, which is also useful when a cytotoxicity test is performed.

Moreover, in the present technology, the morphological change amount of a cell can be quantified in a non-staining manner, and a differentiation process of a cell and an activation process to a specific stimulus can be measured, which is useful for evaluating differentiation of a cell and activity state thereof. In addition, a difference in cell type can also be identified, which is useful for screening of a specific cell (for example, a differentiated cell, a cancer cell, or the like).

(2) Imaging control unit 122

The information processing device 12 according to the present technology may include the imaging control unit 122 as necessary. The imaging control unit 122 determines an imaging interval and an imaging range of the imaging device 11 described above, and causes the imaging device 11 to image an imaged image.

Specifically, for example, the imaging control unit 122 can perform control such that an imaging interval (interval of each frame) of an imaged image is one second or less (one frame/second or more). The motion of a cell regarding an intracellular metabolic state is faster than movement or the like of the cell. Therefore, it is difficult to capture the movement of the cell regarding the intracellular metabolic state when the imaging interval is less than one frame/second. Therefore, in the present technology, 10 frames/second or more is preferable, 20 frames/second or more is more preferable, and 27 frames/second or more is still more preferable.

The range of a field of the imaging device 11 is not particularly limited, and can be freely set appropriately, for example, can be set to a range including one cell, a range including a cell group, or the entire field of the imaging device 11. Furthermore, the imaging control unit 122 may control the imaging device 11 using an image recognition result by the analysis unit 121. For example, the imaging control unit 122 can control an imaging range of the imaging device 11 so as to image a specific cell or cell group detected by the analysis unit 121.

(3) Image acquisition unit 123

The information processing device 12 according to the present technology may include the image acquisition unit 123 as necessary. The image acquisition unit 123 acquires an imaged image. Furthermore, the image acquisition unit 123 can acquire an imaged image directly from the imaging device 11 or via a storage or a network. Then, the image acquisition unit 123 supplies the acquired imaged image to the analysis unit 121.

(4) Display control unit 124

The information processing device 12 according to the present technology may include the display control unit 124 as necessary. The display control unit 124 outputs the result of the evaluation of the intracellular metabolic state, the morphological change amount, information regarding these, and the like provided from the analysis unit 121 to a display or the like, and presents these to a user.

Furthermore, the display control unit 124 can display an image in which information regarding the intracellular metabolic state is superimposed on an imaged image, and for example, can display a cell in color according to a result of the evaluation (for example, ATP concentration and the like). Note that the superimposed image may be generated for a plurality of imaged images having different imaging times, or may be generated by extracting only a cell or a cell group specified by the analysis unit 121.

(5) Hardware configuration of information processing device 12

The functional configuration of the information processing device 12 described above can be achieved by a hardware configuration described below.

Fig. 2 is a schematic diagram illustrating an example of a hardware configuration of the information processing device 12 according to the present technology. As illustrated in Fig. 2, the information processing device 12 includes, for example, a central processing unit (CPU) 101, a graphic processing unit (GPU) 102, a memory 103, a storage 104, and an input/output unit (I/O) 105 as a hardware configuration. These are connected to one another by a bus 106.

The CPU 101 controls another configuration according to a program stored in the memory 103, performs data processing according to the program, and stores a processing result in the memory 103. The CPU 101 can be, for example, a microprocessor or the like.

The GPU 102 executes image processing under control of the CPU 101. The CPU 101 can cause the GPU 102 to execute parallel arithmetic processing to extract a feature amount and the like at high speed. The GPU 102 can be, for example, a microprocessor or the like.

The memory 103 stores a program and data executed by the CPU 101. The memory 103 can be, for example, a random access memory (RAM) or the like.

The storage 104 stores a program and data. The storage 104 can be, for example, a hard disk drive (HDD), a solid state drive (SSD), or the like.

The input/output unit (I/O) 105 receives an input to the information processing device 12 and supplies an output of the information processing device 12 to the outside. The input/output unit 105 includes an input device such as a keyboard or a mouse, an output device such as a display, and a connection interface such as a network.

Note that in the present technology, the hardware configuration of the information processing device 12 is not limited to the above-described one, and may be any one capable of achieving the functional configuration of the information processing device 12. Furthermore, in the present technology, a part or the whole of the hardware configuration may be present on a network.

3. Information processing method

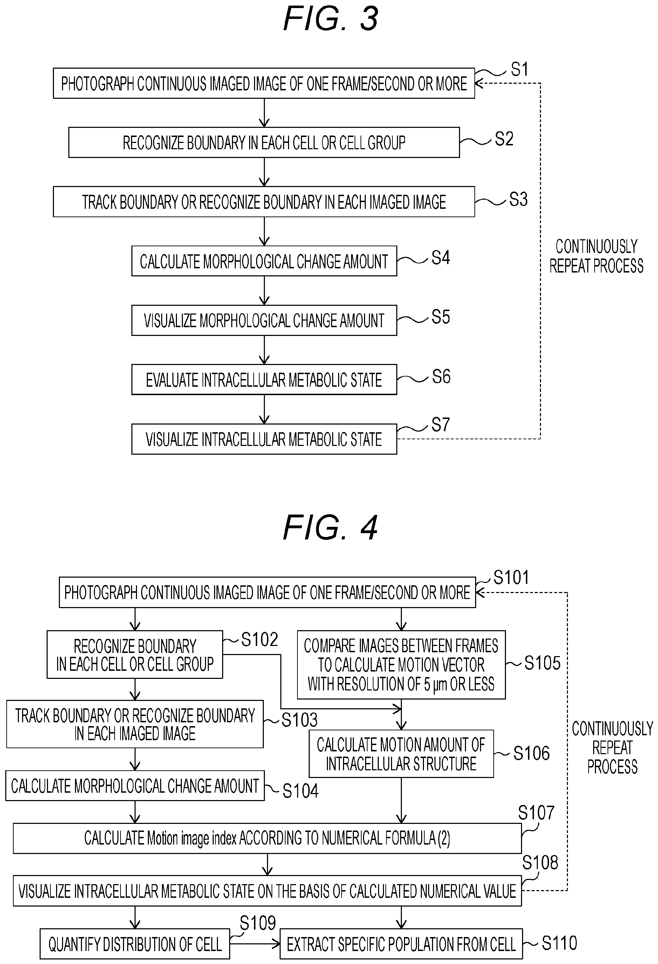

The information processing method according to the present technology performs at least an analysis procedure. A specific method performed in the analysis procedure is the same as the analysis procedure performed by the analysis unit 121 of the information processing device 12 described above, and therefore description thereof will be omitted here. Hereinafter, an information processing example using the information processing method according to the present technology will be described in detail with reference to Figs. 3 and 4.

(1) Information processing example 1

Fig. 3 is a process chart illustrating the information processing example 1 in the information processing method according to the present technology.

In the information processing example 1, first, the imaging device 11 photographs a continuous imaged image (moving image) of one frame/second or more (S1). This photographing is controlled by the imaging control unit 122. Furthermore, the imaged image photographed is acquired by the image acquisition unit 123. Next, the analysis unit 121 recognizes a boundary in each cell or cell group from the imaged image supplied from the image acquisition unit 123 (S2). Specifically, for example, the analysis unit 121 recognizes a boundary between a cell area and a non-cell area in each cell or cell group present in a range designated by a user. Thereafter, the analysis unit 121 tracks a boundary in the cell or the cell group or recognizes a boundary in each imaged image (S3). Then, the analysis unit 121 calculates the morphological change amount by the method described above (S4).

Thereafter, the analysis unit 121 visualizes the morphological change amount (S5). The display control unit 124 presents a result of the visualization to a user. Next, the analysis unit 121 evaluates an intracellular metabolic state by the method described above (S6). Thereafter, the analysis unit 121 color-codes the inside of each cell according to a result of the evaluation, and visualizes the evaluated intracellular metabolic state (S7). The display control unit 124 presents a result of the visualization to a user as color display.

Then, in the information processing example 1, a process from step S1 to step S7 is continuously repeated until arbitrary time or number of times set by the analysis unit 121 or a user is reached.

(2) Information processing example 2

Fig. 4 is a process chart illustrating the information processing example 2 in the information processing method according to the present technology.

In the information processing example 2, first, the imaging device 11 photographs a continuous imaged image (moving image) of one frame/second or more (S101). This photographing is controlled by the imaging control unit 122. Furthermore, the imaged image photographed is acquired by the image acquisition unit 123. Next, the analysis unit 121 recognizes a boundary in each cell or cell group from the imaged image supplied from the image acquisition unit 123 (S102). Specifically, for example, the analysis unit 121 recognizes a boundary between a cell area and a non-cell area in each cell or cell group present in a range designated by a user. Thereafter, the analysis unit 121 tracks a boundary in the cell or the cell group or recognizes a boundary in each imaged image (S103). Then, the analysis unit 121 calculates the morphological change amount by the method described above (S104).

Meanwhile, in parallel with this, after step S101, the analysis unit 121 compares images between frames to calculate a motion vector with a resolution of 5 μm or less (S105). Thereafter, the motion amount of the intracellular structure is calculated by the method described above (S106). Note that in the present technology, as illustrated in Fig. 4, the motion amount of the intracellular structure may be calculated after the process passes through step S102 without passing through step S105 (S106).

After the morphological change amount and the motion amount of the intracellular structure are calculated in steps S104 and S106, respectively, a Motion image index is calculated according to the above numerical formula (2) (S107). In the information processing example 2, the area change amount of a cell is used as the morphological change amount 1, the peripheral length change amount of a cell is used as the morphological change amount 2, and an average of lengths of intracellular motion vectors is used as the motion amount of the intracellular structure in numerical formula (2). However, the present technology is not limited thereto.

Then, the analysis unit 121 color-codes the inside of each cell according to a result in step S107, and visualizes the evaluated intracellular metabolic state (S108). The display control unit 124 presents a result of the visualization to a user as color display.

Then, in the information processing example 2, a process from step S101 to step S108 is continuously repeated until arbitrary time or number of times set by the analysis unit 121 or a user is reached.

Furthermore, in the information processing example 2, after step S108, the analysis unit 121 further quantifies the distribution of the cell (S109). This quantification is performed using, for example, a histogram, a whisker box plot, or the like. Then, after step S108, the analysis unit 121 further extracts a specific population from the cell (S110). After extracting the specific population, the analysis unit 121 can mask the population and display an image. Note that in the present technology, as illustrated in Fig. 4, after step S109, a specific population may be extracted (S110).

EXAMPLES

Hereinafter, the present technology will be described in more detail on the basis of Examples. Note that Examples described below exemplify representative Examples of the present technology, and the scope of the present technology is not narrowly interpreted by Examples.

<Example 1: quantification of morphological change>

An image (moving image) of an HL60 cell that had been differentiated and stimulated by adding 1% DMSO and performing culture for one week was imaged over time with a phase difference microscope under conditions that magnification of an objective lens was 60 times, an imaging interval was 27 frames/second, and imaging time was 60 seconds. Fig. 5 is an image illustrating cell boundary recognition and tracking according to Example 1. As illustrated in Fig. 5, in the first image (frame), a cell boundary was recognized by machine learning based object recognition, and a solid line (white line) was drawn at the boundary. Thereafter, the cell boundary was tracked by matching the drawn solid line to a next image. Then, a cell area in the continuous image was tracked by repeating matching to the continuous image.

Thereafter, the area and the peripheral length were calculated from the tracked cell area, and an average of the areas, an average of the peripheral lengths, and change amounts thereof in the continuous image were calculated. Then, the change amount with respect to the average of the areas or peripheral lengths was calculated as the morphological change amount (area change amount and peripheral length change amount). This was used as Control. By a similar method, as for a case where 100 μM of a mitochondrial unconjugating agent, carbonyl cyanide m-chlorophenylhydrazone (also simply referred to as "CCCP" here), which is a drug that inhibits intracellular metabolism, was added, the morphological change amount was calculated similarly. The upper drawing of Fig. 6 is a drawing-substituting graph illustrating an area change amount and a peripheral length change amount according to Example 1, and the lower drawing of Fig. 6 is an image illustrating cell boundary recognition and tracking in Control and in a case where CCCP is added according to Example 1. As illustrated in the drawing-substituting graph as the upper drawing of Fig. 6, in a case where 100 μM CCCP was added, a significant decrease was confirmed in both the area change amount and the peripheral length change amount.

<Example 2: inhibition of intracellular metabolism>

By a similar method to Example 1 described above, the morphological change amounts (area change amounts and peripheral length change amounts) in a case where CCCP was added at concentrations of 0.01 μM, 0.1 μM, 1 μM, and 10 μM were calculated, respectively. Furthermore, a motion vector under similar conditions was calculated at an interval of about 1 μm, and a spatiotemporal average of the motion amounts (motion speeds) per unit time for each cell was calculated to create a color map. The upper drawing of Fig. 7 is a drawing-substituting graph illustrating the motion amount (vector analysis), the area change amount, and the peripheral length change amount of an intracellular structure according to Example 2, and the lower drawing of Fig. 7 is a color map of a cell including CCCP according to Example 2. The lower drawing of Fig. 7 illustrates the created color map for each CCCP concentration.

As illustrated in the drawing-substituting graph as the upper drawing of Fig. 7, a CCCP concentration-dependent decrease was confirmed in the motion amount (vector analysis) of the intracellular structure, the area change amount, and the peripheral length change amount. Therefore, it was suggested that these parameters were closely related to the intracellular metabolic state.

<Example 3 Correlation with ATP concentration>

The motion amount of the intracellular structure and the morphological change amount (area change amount and peripheral length change amount) were measured by a similar method to Examples 1 and 2 described above. Thereafter, the cell was disrupted, and the ATP concentration was measured. CellTiter-glo manufactured by Promega Corporation was used to measure the ATP concentration. Fig. 8 is a drawing-substituting graph illustrating the motion amount (vector analysis), the area change amount, and the peripheral length change amount of an intracellular structure according to Example 3.

As illustrated in the drawing-substituting graph of Fig. 8, all of the motion amount of the intracellular structure, the area change amount, and the peripheral length change amount were significantly correlated with the ATP concentration. Furthermore, a Motion image index was calculated according to following numerical formula (3) from these results, and weighting with each correlation coefficient was performed.

[Numerical Formula 3]

$$\text{Motion image index} = 0.52 \times \text{area change amount} + 0.56 \times \text{peripheral length change amount} + 0.77 \times \text{motion amount of intracellular structure} \quad \ldots (3)$$

Fig. 9 is a drawing-substituting graph illustrating a correlation between an ATP concentration and a Motion Image Index according to Example 3. As illustrated in the drawing-substituting graph of Fig. 9, the Motion image index calculated using the morphological change amount (area change amount and peripheral length change amount) and the motion amount of the intracellular structure strongly correlated with the ATP concentration (correlation coefficient R = 0.79). Therefore, it was found that by using the motion amount of the intracellular structure in the evaluation, it was possible to more accurately evaluate a change in intracellular state.

<Example 4: Involvement of cytoskeleton>

By a similar method to Example 1 described above, the morphological change amount (area change amount and peripheral length change amount) in a case where 200 nM of Cytochalasin D, which is an actin polymerization inhibitor, was added. The upper drawing of Fig. 10 is a drawing-substituting graph illustrating an area change amount and a peripheral length change amount according to Example 4, and the lower drawing of Fig. 10 is an image illustrating cell boundary recognition and tracking in Control and in a case where Cytochalasin D is added according to Example 4.

As illustrated in the drawing-substituting graph as the upper drawing of Fig. 10, by adding an actin polymerization inhibitor, a significant decrease was confirmed in both the area change amount and the peripheral length change amount. Therefore, it was suggested that the cytoskeleton was involved in the morphological change amount.

<Example 5: Involvement of ATP-dependent motor protein>

By a similar method to Examples 1 and 2 described above, the morphological change amount (area change amount and peripheral length change amount) and the motion amount of the intracellular structure were calculated in a case where 500 μM of Monastrol, which is a kinesin inhibitor as a motor protein that acts as power in a cell using ATP, was added, and in a case where 500 μM of Ciliobrevin D, which is a dynein inhibitor as a motor protein that acts as power in a cell using ATP, was added. Fig. 11 is a drawing-substituting graph illustrating the motion amount (vector analysis) of the intracellular structure, the area change amount, and the peripheral length change amount according to Example 5.

As illustrated in the drawing-substituting graph of Fig. 11, by adding these inhibitors, a significant decrease was confirmed in all of the motion amount of the intracellular structure, the area change amount, and the peripheral length change amount. Therefore, it was suggested that the ATP-dependent motor protein was involved in the morphological change amount and the motion amount of the intracellular structure.

Note that the present technology can have the following configurations.

(1) An information processing device including at least an analysis unit for calculating a morphological change amount of a cell per unit time from an image obtained by imaging the cell, and evaluating an intracellular metabolic state on the basis of the morphological change amount of the cell per unit time.

(2) The information processing device according to (1), in which the morphological change amount is based on a change amount of the area of the cell per unit time.

(3) The information processing device according to (1) or (2), in which the morphological change amount is based on a change amount of the peripheral length of the cell per unit time.

(4) The information processing device according to any one of (1) to (3), in which the morphological change amount is based on a change amount of roundness of the cell per unit time.

(5) The information processing device according to any one of (1) to (4), in which the morphological change amount is based on a change amount of the volume of the cell per unit time.

(6) The information processing device according to any one of (1) to (5), in which the morphological change amount is based on a change amount of movement of a structure generated by the cell per unit time.

(7) The information processing device according to any one of (1) to (6), in which the analysis unit further calculates a motion amount of an intracellular structure per unit time from an image obtained by imaging the cell, and uses the movement amount of the intracellular structure per unit time in the evaluation.

(8) The information processing device according to any one of (1) to (7), in which the analysis unit further color-codes the inside of each cell according to a result of the evaluation.

(9) The information processing device according to (8), in which the analysis unit further quantifies a distribution of the cell after the color-coding.

(10) The information processing device according to (8) or (9), in which the analysis unit further extracts a specific population from the cell after the color-coding.

(11) The information processing device according to any one of (1) to (10), in which the analysis unit calculates an intracellular ATP concentration on the basis of the morphological change amount of the cell per unit time.

(12) The information processing device according to any one of (1) to (11), in which the analysis unit further sets a threshold to a value of the evaluation and determines whether the cell is a living cell or a dead cell.

(13) The information processing device according to any one of (1) to (12), in which the cell is a floating cell.

(14) The information processing device according to (13), in which the floating cell includes at least one selected from a group consisting of a white blood cell, a white blood cell precursor cell, a white blood cell-derived tumor cell, and a blood circulating tumor cell.

(15) An information processing system including at least:

an imaging device; and
an information processing device including at least an analysis unit for calculating a morphological change amount of a cell per unit time from an image obtained by imaging the cell with the imaging device, and evaluating an intracellular metabolic state on the basis of the morphological change amount of the cell per unit time.

(16) An information processing method including at least
an analysis procedure for calculating a morphological change amount of a cell per unit time from an image obtained by imaging the cell, and evaluating an intracellular metabolic state on the basis of the morphological change amount of the cell per unit time.

(17) The information processing method according to (16), in which in the analysis procedure, a motion amount of an intracellular structure per unit time is further calculated from an image obtained by imaging the cell, and the motion amount of the intracellular structure per unit time is used in the evaluation.

REFERENCE SIGNS LIST

1       Information processing system
11      Imaging device
12      Information processing device
121     Analysis unit
122     Imaging control unit
123     Image acquisition unit
124     Display control unit

101     CPU
102     GPU
103     Memory
104     Storage
105     Input/output unit (I/O)
106     Bus

**Claims**

1.  An information processing device comprising at least an analysis unit for calculating a morphological change amount of a cell per unit time from an image obtained by imaging the cell, and evaluating an intracellular metabolic state on a basis of the morphological change amount of the cell per unit time.

2.  The information processing device according to claim 1, wherein the morphological change amount is based on a change amount of an area of the cell per unit time.

3.  The information processing device according to claim 1, wherein the morphological change amount is based on a change amount of a peripheral length of the cell per unit time.

4.  The information processing device according to claim 1, wherein the morphological change amount is based on a change amount of roundness of the cell per unit time.

5.  The information processing device according to claim 1, wherein the morphological change amount is based on a change amount of a volume of the cell per unit time.

6.  The information processing device according to claim 1, wherein the morphological change amount is based on a change amount of movement of a structure generated by the cell per unit time.

7.  The information processing device according to claim 1, wherein the analysis unit further calculates a motion amount of an intracellular structure per unit time from an image obtained by imaging the cell, and uses the motion amount of the intracellular structure per unit time in the evaluation.

8.  The information processing device according to claim 1, wherein the analysis unit further color-codes an inside of each cell according to a result of the evaluation.

9.  The information processing device according to claim 8, wherein the analysis unit further quantifies a distribution of the cell after the color-coding.

10. The information processing device according to claim 8, wherein the analysis unit further extracts a specific population from the cell after the color-coding.

11. The information processing device according to claim 1, wherein the analysis unit calculates an intracellular ATP concentration on a basis of the morphological change amount of the cell per unit time.

12. The information processing device according to claim 1, wherein the analysis unit further sets a threshold to a value of the evaluation and determines whether the cell is a living cell or a dead cell.

13. The information processing device according to claim 1, wherein the cell is a floating cell.

14. The information processing device according to claim 13, wherein the floating cell includes at least one selected from a group consisting of a white blood cell, a white blood cell precursor cell, a white blood cell-derived tumor cell, and a blood circulating tumor cell.

15. An information processing system comprising at least:

    an imaging device; and
    an information processing device including at least an analysis unit for calculating a morphological change amount of a cell per unit time from an image obtained by imaging the cell with the imaging device, and evaluating an intracellular metabolic state on a basis of the morphological change amount of the cell per unit time.

16. An information processing method comprising at least
an analysis procedure for calculating a morphological change amount of a cell per unit time from an image obtained by imaging the cell, and evaluating an intracellular metabolic state on a basis of the morphological change amount of the cell per unit time.

17. The information processing method according to claim 16, wherein in the analysis procedure, a motion amount of an intracellular structure per unit time is further calculated from an image obtained by imaging the cell, and the motion amount of the intracellular structure per unit time is used in the evaluation.

# FIG. 1

# FIG. 2

# FIG. 3

PHOTOGRAPH CONTINUOUS IMAGED IMAGE OF ONE FRAME/SECOND OR MORE — S1

RECOGNIZE BOUNDARY IN EACH CELL OR CELL GROUP — S2

TRACK BOUNDARY OR RECOGNIZE BOUNDARY IN EACH IMAGED IMAGE — S3

CALCULATE MORPHOLOGICAL CHANGE AMOUNT — S4

VISUALIZE MORPHOLOGICAL CHANGE AMOUNT — S5

EVALUATE INTRACELLULAR METABOLIC STATE — S6

VISUALIZE INTRACELLULAR METABOLIC STATE — S7

CONTINUOUSLY REPEAT PROCESS

# FIG. 4

PHOTOGRAPH CONTINUOUS IMAGED IMAGE OF ONE FRAME/SECOND OR MORE — S101

RECOGNIZE BOUNDARY IN EACH CELL OR CELL GROUP — S102

COMPARE IMAGES BETWEEN FRAMES TO CALCULATE MOTION VECTOR WITH RESOLUTION OF 5 μm OR LESS — S105

TRACK BOUNDARY OR RECOGNIZE BOUNDARY IN EACH IMAGED IMAGE — S103

CALCULATE MOTION AMOUNT OF INTRACELLULAR STRUCTURE — S106

CALCULATE MORPHOLOGICAL CHANGE AMOUNT — S104

CALCULATE Motion image index ACCORDING TO NUMERICAL FORMULA (2) — S107

VISUALIZE INTRACELLULAR METABOLIC STATE ON THE BASIS OF CALCULATED NUMERICAL VALUE — S108

QUANTIFY DISTRIBUTION OF CELL — S109

EXTRACT SPECIFIC POPULATION FROM CELL — S110

CONTINUOUSLY REPEAT PROCESS

## FIG. 5

## FIG. 6

AREA CHANGE AMOUNT

PERIPHERAL LENGTH CHANGE AMOUNT

* P < 0.05

## FIG. 7

MOTION AMOUNT OF INTRACELLULAR STRUCTURE

AREA CHANGE AMOUNT

PERIPHERAL LENGTH CHANGE AMOUNT

## FIG. 8

MOTION AMOUNT OF
INTRACELLULAR STRUCTURE

R=0.77

AREA CHANGE AMOUNT

R=0.52
p=0.021

PERIPHERAL LENGTH
CHANGE AMOUNT

R=0.56
p=0.011

## FIG. 9

R=0.79

## FIG. 10

AREA CHANGE AMOUNT

PERIPHERAL LENGTH
CHANGE AMOUNT

* P < 0.05

0 sec                Time                60 sec

Control

Cytochalasin D

# FIG. 11

KINESIN INHIBITOR: Monastrol 500 μM
DYNEIN INHIBITOR: Ciliobrevin D 500 μM

* P < 0.05

MOTION AMOUNT OF
INTRACELLULAR STRUCTURE

AREA CHANGE AMOUNT

PERIPHERAL LENGTH
CHANGE AMOUNT

## INTERNATIONAL SEARCH REPORT

International application No.

PCT/JP2018/005946

### A. CLASSIFICATION OF SUBJECT MATTER

Int.Cl. C12M1/34(2006.01)i, C12M1/00(2006.01)i, G01N33/48(2006.01)i, G01N33/49(2006.01)i, G06T7/00(2017.01)i

According to International Patent Classification (IPC) or to both national classification and IPC

### B. FIELDS SEARCHED

Minimum documentation searched (classification system followed by classification symbols)

Int.Cl. C12M1/34, C12M1/00, G01N33/48, G01N33/49, G06T7/00

Documentation searched other than minimum documentation to the extent that such documents are included in the fields searched

| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

JSTPlus/JMEDPlus/JST7580(JDreamIII)
CAplus/MEDLINE/EMBASE/BIOSIS(STN)

### C. DOCUMENTS CONSIDERED TO BE RELEVANT

| Category* | Citation of document, with indication, where appropriate, of the relevant passages | Relevant to claim No. |
|---|---|---|
| X | WO 2017/002300 A1 (SONY CORPORATION) 05 January 2017, claims, examples & JP 2017-16628 A, claims, examples | 1, 6, 7, 11, 15–17 |
| Y | | 2–14 |
| X | JP 2005-521126 A (REIFY CORPORATION) 14 July 2005, claims & US 2003/0185450 A1, claims & WO 2003/077552 A1 & EP 1486067 A | 1, 6, 7, 15–17 |
| Y | | 2–14 |
| Y | WO 2011/099565 A1 (SYSMEX CORPORATION) 18 August 2011, claims, paragraph [0075], examples & JP 11-99565 A1 | 2–14 |
| A | | 1, 15–17 |

☐ Further documents are listed in the continuation of Box C.  ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 16 May 2018 (16.05.2018) | 29 May 2018 (29.05.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**EP 3 591 032 A1**

**Patent documents cited in the description**

- JP 2017016628 A